(19) Europäisches Patentamt
European Patent Office
Office européen des brevets

(11) Publication number: **0 194 781 B1**

(12) # EUROPEAN PATENT SPECIFICATION

(45) Date of publication of the patent specification: **24.08.88**

(51) Int. Cl.⁴: **C 07 C 19/08**, C 07 C 17/20

(21) Application number: **86301380.1**

(22) Date of filing: **26.02.86**

(54) **Process for preparing alpha, omega-haloperfluoroalkanes.**

(30) Priority: **26.02.85 IT 1965285**

(43) Date of publication of application: **17.09.86 Bulletin 86/38**

(45) Publication of the grant of the patent: **24.08.88 Bulletin 88/34**

(84) Designated Contracting States: **BE DE FR GB IT NL**

(56) References cited: **US - A - 3 381 043**

(73) Proprietor: **AUSIMONT S.p.A., 31, Foro Buonaparte, I-20121 Milano (IT)**

(72) Inventor: **Caporiccio, Gerardo, 13, Via e. Filiberto, I-20149 Milan (IT)**
Inventor: **Bargigia, Gianangelo, 47, Via Federico Chopin, I-20141 Milan (IT)**
Inventor: **Tonelli, Claudio, 25, Via Gino Valagussa, I-20049 Concorezzo Milan (IT)**
Inventor: **Tortelli, Vito, 51, Piazza Aspromonte, I-20131 Milan (IT)**

(74) Representative: **Whalley, Kevin et al, MARKS & CLERK 57/60 Lincoln's Inn Fields, London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to a process for preparing α,ω-haloperfluoroalkanes.

Known in the art and industrially produced are haloperfluoroalkanes, which are utilized in many technological fields depending on their chemical and chemical-physical characteristics.

A limitation of the use thereof is caused by their low boiling points, as is apparent from the following Table.

| Chemical formula | Boiling point (°C at 1 at.abs.) | Examples of fields of use |
|---|---|---|
| $CCl_3F$ | +23.8 | Aerosol, foaming of polyurethanes |
| $CCl_2F_2$ | - 29.8 | Aerosol, refrigeration, foaming of polyolefins |
| $CHClF_2$ | - 40.8 | Refrigeration |
| $C_2Cl_3F_3$ | +47.6 | Solvent, foaming of resins |
| $C_2Cl_2F_4$ | + 3.6 | Aerosol, foaming of polyolefins |
| $C_2ClF_5$ | - 39.1 | Refrigeration |
| $CBrF_3$ | - 57.8 | Flame extinguishing agent |
| $C_2Br_2F_4$ | +46.7 | Flame extinguishing agent |

These products, therefore, are utilizable only in fields where high boiling points are not required.

The synthesis methods known so far for preparing products exhibiting a higher molecular weight and as a consequence a higher boiling point, having general formula $Cl(C_2F_4)_nCl$ with n greater than or equal to 2, are not sufficiently selective or unsuited for commercial-scale utilization.

For example in U.S. Patent No. US-A-3 381 043 it is stated that the addition reaction of chlorine to $C_2F_4$ is too strong to be capable of giving rise to telomerization. In practice that means that the reaction stops at the first group $ClC_2F_4Cl$ and does not permit to introduce further units $C_2F_4$ between the two chlorine atoms in order to obtain the higher homologous products.

The above cited patent describes a process for preparing by telomerization the groups higher than $ClC_2F_4Cl$ by operating in the presence of $PCl_5$. However, the reaction with $PCl_5$, owing to the fact that this product is solid, requires the presence of solvents to promote a better contact between the reagents. Generally, $CCl_4$ is used as a solvent.

In this case, however, the degree of utilization of the systems employed for the synthesis is reduced, with consequent considerable increase in the product costs.

Furthermore, according to said patent, the obtained products can be separated only by using gas chromatographic techniques. In fact, a fractional distillation appears impossible for products $Cl(C_2F_4)_nCl$ wherein n is less than or equal to 4 because of the formation of azeotropes between $Cl(C_2F_4)_3Cl$ and $CCl_4$ employed as a solvent. Only the fractions having n greater than 4 can be obtained by means of the distillation technique. However, the products with n less than or equal to 4 are the most interesting from a practical viewpoint.

This process for the preparation of telomers is of no practical interest from an industrial aspect because it is not practical to use gas chromatographic techniques for preparing considerable amounts of the individual telomers with n less than or equal to 4.

It has now surprisingly been found that it is possible to prepare the products mentioned hereinabove, without operating in the presence of $PCl_5$ or of solvents, by means of the process according to the present invention, by using, as a telogen, a derivative of $C_2F_4$ having, as end groups, at least one iodine atom, the other valence being saturated by a halogen atom of any kind, provided it is different from fluorine.

In fact it has been found that if $C_2F_4$ has both valences saturated with Br, the telomerization occurs with great difficulty, as a result of which the yields of telomers are extremely low.

The present invention provides a process for preparing tetrafluoroethylene telomers having the general formula:

$$X(C_2F_4)_nY$$

wherein n is an integer from 2 to 6, extremes included, preferably from 2 to 4, and X and Y, which may be the same as or different from each other, are Cl or Br. The process comprises the steps of preparing a tetrafluoroethylene telomer having an end group containing iodine, the other end group of the chain containing a halogen such as Cl, Br or I, and subsequently reacting with $Cl_2$ or with $Br_2$.

The products obtainable according to the present invention are more stable than analogous products, preparable for example by addition of halogen (Cl or Br) to an olefinic double bond, because the latter have the halogen atoms bound to adjacent carbon atoms, that is in a favourable position for easily undergoing a dehalogenation with metals, for example zinc.

The process employed for preparing the products of the present invention consists in subejcting $C_2F_4$ to telomerization by using $Z\text{-}CF_2CF_2\text{-}I$ as a telogen according to the reaction:

$$Z\text{-}CF_2CF_2\text{-}I + (n-1)\ CF_2 = CF_2 \rightarrow Z\text{-}(CF_2CF_2)_n\text{-}I$$

wherein Z is Cl, Br, I and n has the meaning indicated hereinabove,

and subsequently effecting a halogenation reaction with $Cl_2$ or $Br_2$ of $Z\text{-}(CF_2CF_2)_n\text{-}I$ to yield $X\text{-}(CF_2CF_2)_n\text{-}Y$

wherein n, X and Y are the same as indicated hereinbefore.

The reaction according to the above scheme is carried out by telomerization of $C_2F_4$ using, as a telogen, 1,2-di-iodotetrafluoroethane or

1-bromo-2-iodotetrafluoroethane or
1-chloro-2-iodotetrafluoroethane.

The latter are preparable in the pure state by reaction of iodine, iodine bromides or iodine chlorides with $C_2F_4$, employing $C_2F_4$/halogenating agent molar ratios equal to or lower than 1. Subsequently, the telomerization with $C_2F_4$, as indicated hereinafter, is carried out.

According to an alternative method, telomers Z-$(CF_2F_2)_n$-I are directly preparable by using an excess of $C_2F_4$ with respect to the iodine halides or to the iodine and by successively reacting the resulting telomers with $Cl_2$ or $Br_2$ to obtain the products of the invention.

The telomerization may be conducted by means of heat at temperatures generally ranging from 150° to 250°C, or in the presence of peroxides at temperatures of from 45° to 250°C. The utilizable peroxides include peroxydicarbonates, such as
bis-(4-tert.-butylcyclohexyl)peroxydicarbonate or
bis-(4-alkylcyclohexyl)peroxydicarbonate, in which the alkyl contains from 2 to 8 carbon atoms. There are employable also redox systems such as for example $Cu^+$/ethanolamine, $Cu^+$/diethanolamine.

According to the present invention, the subsequent reaction for introducing chlorine or bromine atoms is effected by directly treating
α,ω-diiodoperfluoroalkanes,
α,ω-bromoiodoperfluoralkanes and
α,ω-chloroiodoperfluoroalkanes with preferably 0.5 to 10 moles, more preferably from 1 to 5 moles, of elemental $Cl_2$ or $Br_2$ per atom of iodine, at temperatures from 50° to 100°C.

The reaction may be conducted at ambient pressure, or under an autogenous pressure, depending on the employed halogen amount, on the reactor dimensions and on the temperatures, at which the reaction is conducted, generally up to 30 atm. or above; the presence of ultraviolet radiation promotes the reaction so as to make it possible to use lower temperatures, generally from 0° to 50°C.

Examples of products obtained according to the present invention are:

Cl-$(CF_2CF_2)_2$-Cl
Br-$(CF_2CF_2)_2$-Br
Cl-$(CF_2CF_2)_3$-Cl
Cl-$(CF_2CF_2)_3$-Br
Cl-$(CF_2CF_2)_4$-Cl

The advantages deriving from the process of the invention are that there may be obtained a whole class of products through a simple reaction, easily reproduceable on an industrial scale, without the need of employing solvents.

The reaction does not provide by-products because the iodine or the iodine halides are easy to recover and recycle.

The products prepared by the process according to the invention are stable to chemical agents and to heat and exhibit good dielectric properties, as a result of which they can be used as coolants for electrical and electronic devices, also under tension, and as operating fluids for Rankine cycles.

In addition to the properties mentioned above, the products of the invention possess also densities far higher than 1, as a result of which they can be used as dielectric fluids for submerged devices, such as submarine cables.

The invention will be further described with reference to the following illustrative Examples.

*Example 1*

Into a stainless steel AISI autoclave having a capacity of 5 litres, equipped with a magnetic stirrer, there were charged 450 g (0.99 moles) of 1,4-diiodooctafluorobutane, I-$(C_2F_4)_2$-I, obtained by telomerization of $C_2F_4$ with I-$C_2F_4$-I and subsequent fractional distillation.

After having closed and deaerated the autoclave with nitrogen, 360 g (5.1 moles) of chlorine were introduced. The autoclave was heated to150°C during 10 hours under continuous stirring. The autoclave was cooled down, chlorine in excess was removed and the resulting product was washed with an aqueous solution of sodium bisulphite in order to remove traces of oxidants such as $I_2$ or $Cl_2$. The organic layer was then separated, which amounted to 256 g.

Gas chromatographic (GC) analysis (column of 4 m; fluorinated neutral oils Chromosorb W; Hewlett-Packard integrator; 100°C) revealed that the product was $Cl(C_2F_4)_2Cl$ and that it was particularly pure, because by-products were present only in traces. Further two peaks with a higher retention time were observed, the second of which exhibited the same retention time as I-$(C_2F_4)_2$-I which had been utilized as a reagent.

NMR($^{19}F$) analysis (by a Varian XL 200) revealed, in an integration ratio of 1:1, two chemical shifts (δ, $CCl_3F$) respectively at: ppm 119 (-$\overline{CF}_2CF_2$) and ppm 69 ($Cl\overline{CF}_2$-) as a result of which the product was found to have the following structure:

$$ClCF_2CF_2CF_2Cl.$$

The main characteristics of the product were:

| | |
|---|---|
| boiling point | 65°C |
| melting point | –83°C |
| density | 1.65 g/cm³ |
| latent heat of evaporation at boiling temperature | 7.5 kcal/mole |
| surface tension | 18 dynes/cm |
| vapour tension at 22°C | 150 mm Hg. |

*Example 2*

Into a cylindrical reactor (diameter 2 cm), made of glass, equipped with a fritted bottom for gas inlet, connected to an inert gas ($N_2$) line and to a chlorine cylinder and also equipped with two coolers connected in series and maintained at +15°C and –40°C, respectively, 70 g (0.154 moles) of $I(C_2F_4)_2I$ (prepared according to Example 1) were introduced under a slight nitrogen flow.

The nitrogen flow was gradually substituted by a $Cl_2$ flow (0.5 l/h), following by heating up to 140°C; it was observed that the solution turned dark due to release of $I_2$ and that, as time passed, it tended to

assume a straw yellow colour due to the forming of interhalogen compounds. At regular intervals of time samples were drawn for GC analysis. After 8 hours 60% of the reagent was converted: for 8/10 to intermediate product $Cl(CF_2CF_2CF_2CF_2)I$, boiling point: 107°C (recognized through ($^{19}F$) NMR analysis: (δ, $CFCl_3$) at ppm 69; 119; 112; 59, respectively) and for the remaining 2/10 to final product $Cl(C_2F_4)_2Cl$. After a further 8 hours, the conversion reached 85%, of which 45% was $Cl(C_2F_4)_2I$ and 55% $Cl(C_2F_4)_2Cl$.

By carrying out the reaction for an additional 8 hours, a conversion of 95% to $Cl(C_2F_4)_2Cl$ was obtained.

Example 3

332 g (0.60 moles) of $I(C_2F_4)_3I$, prepared by telomerization, as indicated in Example 1, and 230 g (3.2 moles) fo chlorine were reacted in the same autoclave and in the same manner as described in Example 1. Heating to 150°C was carried out during 10 hours.

Following the procedure described in Example 1 there were isolated 205 g of a product which, on GC analysis, exhibited a peak at 6.4 min. corresponding to 98% of the total, the remaining 2% being recognized as consisting of $I(C_2F_4)_3Cl$ and unreacted reagent $I(C_2F_4)_3I$. ($^{19}F$) NMR analysis revealed that the product had the following chemical shifts (δ, $CCl_3F$): $Cl\overline{CF_2}$ (ppm 69); $ClCF_2\overline{CF_2}$ (ppm 119); $ClCF_2CF_2\overline{CF_2}$ (ppm 121) corresponding to formula $Cl(C_2F_4)_3Cl$.

The product exhibited the following main characteristics:

| | |
|---|---|
| boiling point | 112°C |
| melting point | -61°C |
| density | 1.74 g/cm$^3$ |
| latent heat of evaporation at boling temperature | 11 kcal/mole |
| surface tension | 21 dynes/cm |
| vapour tension at 20°C | 18 mm Hg. |

*Example 4*

The electrical characteristics of $Cl(C_2F_4)_2Cl$ obtained in Example 1 were evaluated.

By means of a Tettex 2821 apparatus with cell for fluids, the dielectric constant and the dissipation factor (tgδ) were determined.

As a function of potential difference ΔV (at 50 Hz), the following results were obtained:

| ΔV | dielectric constant (relating to air) | tgδ |
|---|---|---|
| 100 | 2.14 | 0.0043 |
| 500 | 2.14 | 0.0045 |
| 1000 | 2.14 | 0.0046 |
| 2000 | 2.14 | 0.0044 |

By means of a Hewlett Packard apparatus, type 4329 A, the volume resistivity was determined on the same fluid, in direct current; such resistivity, under a ΔV of 1000 volts, being equal to $0.5 \cdot 10^{14}$.

*Example 5*

87 g of $I(C_2F_4)_3Cl$ (0.16 moles) were charged into an Inconel 250-ml autoclave. After having closed and deaerated the autoclave with $N_2$, 127 g (0.793 moles) of $Br_2$ were introduced. Heating to 150°C was carried out during 8 hours under continuous stirring. After cooling, the product was washed, in order, with water, 30% KOH, and sodium bisulphite at 10%.

The organic layer (70 g) was separated. The GC analysis revealed a main peak (99%) and another peak equal to 1%, the retention time thereof corresponding to the unreacted starting $I(C_2F_4)_3Cl$.

From ($^{19}F$) NMR analysis there were obtained three peaks of equal intensity (δ, $CCl_3F$); $Br\overline{CF_2}$ (ppm 64); $BrCF_2\overline{CF_2}$ (ppm 117); $BrCF_2CF_2\overline{CF_2}$ (ppm 121), as a result of which the product was found to have the following formula:

$$BrCF_2CF_2CF_2CF_2CF_2CF_2Br.$$

*Example 6*

Following the procedure described in Example 2, 70 g (0.154 moles) of $I(C_2F_4)_2I$ were reacted with chlorine during 8 hours. The resulting product was rectified on an adiabatic column with automatic withdrawing.

25 g (0.06 moles) of the fraction corresponding to $ClCF_2CF_2CF_2CF_2I$ were introduced into an AISI steel small autoclave along with 35 g of bromine (0.22 moles) and treated as described in Example 5.

On the basis of ($^{19}F$) NMR analysis, the resulting product was recognized as $ClCF_2CF_2CF_2CF_2Br$. The chemical shifts were at: ppm 69 for $ClCF_2$; ppm 119 for $ClCF_2\overline{CF_2}$; ppm 117 for $\overline{CF_2}CF_2Br$; ppm 64 for $\overline{CF_2}Br$.

*Example 7*

In order to evaluate the consistency of the α,ω-dichlorooctafluorobutane, obtained as described in Example 1, with plastomers and elastomers, rectangular weighed specimens of polymers were immersed during 72 hours in the above-said liquid maintained at 50°C.

The specimens were dried with filter paper and weighed again. the weight variations are indicated hereinbelow:

| | |
|---|---|
| — polyethylene | +3.9% |
| — polycarbonate | 0.0% |
| — polysulphone | 0.0% |
| — polyvinyl chloride | - 0.1% |
| — polymethylmethacrylate | - 0.2% |
| — polybutylene terephthalate | 0.0% |
| — ABS | +0.3% |
| — nylon 6 | - 0.4% |
| — polypropylene | +1.0% |
| — butadiene-acrylonitrile copolymer | +1.0% |

From the obtained values it is inferable that the consistency of α,ω-dichlorooctafluorobutane with the polymers employed was excellent.

**Claims**

1. A process for preparing tetrafluoroethylene telomers having the general formula:

$$X(C_2F_4)_nY$$

wherein n is an integer from 2 to 6, and X and Y, which may be the same as or different from each other, are Cl or Br, characterized by preparing a tetrafluoroethylene telomer having an end group containing iodine, the other end group containing a halogen, and subsequently reacting the said telomer with chlorine or bromine.

2. A process as claimed in claim 1, characterized in that the molar ratio between the iodurated tetrafluoroethylene telomer and the elemental chlorine or bromine is from 0.5 to 10 moles of $Cl_2$ or $Br_2$ per iodine atom of the telomer.

3. A process as claimed in claim 2, characterized in that the said molar ratio is from 1 to 5.

4. A process as claimed in any of claims 1 to 3, characterized in that the temperature is from 50° to 180°C.

5. A process as claimed in any of claims 1 to 4, characterized in that the halogenation reaction with chlorine or bromine is conducted under the autogenous pressure of chlorine or bromine.

6. A process as claimed in any of claims 1 to 4, characterized in that the halogenation reaction is conducted at ambient pressure.

7. A process as claimed in any of claims 1 to 3, characterized in that the halogenation reaction is conducted in the presence of UV-radiations at a temperature of from 0° to 50°C.

8. A process as claimed in any of claims 1 to 7, characterized in that n is an integer from 2 to 4.

**Patentansprüche**

1. Verfahren zur Herstellung von Tetrafluoroethylentelomeren mit der allgemeinen Formel

$$X(C_2F_4)_nY$$

in welcher n eine ganze Zahl von 2 bis 6 ist und X und Y, die gleich oder verschieden voneinander sein können, Cl oder Br bedeuten, dadurch gekennzeichnet, dass man ein Tetrafluoroethylentelomer mit einer Jod enthaltenden Endgruppe, wobei die andere Endgruppe ein Halogenatom enthält, herstellt und anschliessend dieses Telomer mit Chlor oder Brom umsetzt.

2. Verfahren nach Anspruch 1, dadurch gekennzeichnet, dass das Mol-Verhältnis zwischen dem jodierten Tetrafluoroethylentelomer und dem elementaren Chlor oder Brom von 0,5 bis 10 Mol $Cl_2$ oder $Br_2$ pro Jodatom des Telomers beträgt.

3. Verfahren nach Anspruch 2, dadurch gekennzeichnet, dass das Mol-Verhältnis von 1 bis 5 beträgt.

4. Verfahren nach irgendeinem der Ansprüche 1 bis 3, dadurch gekennzeichnet, dass die Temperatur von 50 bis 180°C beträgt.

5. Verfahren nach irgendeinem der Ansprüche 1 bis 4, dadurch gekennzeichnet, dass die Halogenierung mit Chlor oder Brom unter dem autogenen Druck von Chlor oder Brom erfolgt.

6. Verfahren nach irgendeinem der Ansprüche 1 bis 4, dadurch gekennzeichnet, dass die Halogenierung bei Umgebungsdruck erfolgt.

7. Verfahren nach irgendeinem der Ansprüche 1 bis 3, dadurch gekennzeichnet, dass die Halogenierung in Anwesenheit von UV-Strahlungen bei einer Temperatur von 0 bis 50°C erfolgt.

8. Verfahren nach irgendeinem der Ansprüche 1 bis 7, dadurch gekennzeichnet, dass n eine ganze Zahl von 2 bis 4 ist.

**Revendications**

1. Un procédé de préparation de télomères de tétrafluoroéthylène de formule générale:

$$X(C_2F_4)_nY$$

dans laquelle:

n représente un nombre entier compris entre 2 et 6; et

X et Y qui peuvent être identiques ou différents l'un de l'autre représentent Cl ou Br; caractérisé en ce que l'on prépare un télomère de tétrafluoroéthylène comportant un groupe final contenant de l'iode, l'autre groupe final comportant un halogène, et l'on fait ensuite réagir ce télomère avec du chlore ou du brome.

2. Un procédé selon la revendication 1, caractérisé en ce que le rapport molaire entre le télomère de tétrafluoroéthylène ioduré et le chlore ou le brome élémentaire est compris entre 0,5 et 10 moles de $Cl_2$ ou $Br_2$ par atome d'iode du télomère.

3. Un procédé selon la revendication 2, caractérisé en ce que ce rapport molaire est compris entre 1 et 5.

4. Un procédé selon l'une quelconque des revendications 1 à 3, caractérisé en ce que la température est comprise entre 50° et 180°C.

5. Un procédé selon l'une quelconque des revendications 1 à 4, caractérisé en ce que l'on conduit la réaction d'halogénation en présence de chlore ou de brome sous une pression autogène de chlore ou de brome.

6. Un procédé selon l'une quelconque des revendications 1 à 4, caractérisé en ce que l'on conduit la réaction d'halogénation à la pression ambiante.

7. Un procédé selon l'une quelconque des revendications 1 à 3, caractérisé en ce que l'on conduit la réaction d'halogénation en présence de rayonnement UV, à une température de 0° à 50°C.

8. Un procédé selon l'une quelconque des revendications 1 à 7, caractérisé en ce que n est un nombre entier compris entre 2 et 4.